# EUROPEAN PATENT APPLICATION

(11) **EP 4 353 087 A1**
(43) Date of publication of application: **17.04.2024**
(21) Application number: 22203394.6
(22) Date of filing: 24.10.2022
(51) Int. Cl.: A23L 29/256, A23J 3/14, A23J 3/22, A23L 29/281

(54) **MEAT ANALOGUE PRODUCTS**

(30) Priority: 11.10.2022 US 202263414948 P
(71) Applicant: BK Giulini GmbH, 68526 Ladenburg (DE); Plantible Foods, Inc., San Marcos, California 92069 (US)
(72) Inventor: GRAHN, Irene, 67071 LUDWIIGSHAFEN (DE); GERDES, Annika, 68239 MANNHEIM (DE); SRICHUWONG, Sathaporn, 69115 HEIDELBERG (DE); SPORKA, Radovan, 69117 HEIDELBERG (DE); CILLIERS, Nina, SAN DIEGO, 92117 (US); DAVIS, Huntington, Sacramento, 95747 (US)
(74) Representative: Hoyng Rokh Monegier B.V.

(57) **Abstract**

The composition of the present invention is suitable for use in preparing emulsion-type meat analogue, preferably as a sausage type in a casing, which comprises rubisco protein and kappa-carrageenan in relative amounts between 1:5 to 30:1. Generally, the composition comprises 0.5-30 wt% rubisco and 0.1-15 wt% carrageenan relative the finished meat analogue product, and vegan protein other than rubisco preferably in an amount of 0.5 - 30 wt% relative to the meat analogue product. The meat analogue may contain the composition described in an amount of between 2-40 wt% with 30-90 wt% water and 2-40 wt% plant-based lipid.

## Description

### FIELD OF THE INVENTION

This present invention relates to a dry composition to produce meat analogue products, and to meat analogues obtained with the dry composition.

### BACKGROUND

In recent years, consumers are increasingly trying to limit the amount of meat and other animal-based foods in their diets, because of negative environmental impact of animal farming and health concerns related to meat consumption. These trends create an increasing demand for meat analogue products which completely substitute meat with plant-based ingredients. Consumers expect that such meat analogues should have similar sensorial profiles to the meat product counterparts when consumed at hot and cold stages.

For this reason, several non-animal derived ingredients have been used in the manufacturing of meat analogues to optimize textural properties in order to meet consumer expectations. Meat analogue products are generally compositions comprising a mixture of texturizing ingredients and at least one plant-based protein material to achieve desirable textures for hot and cold conditions. Emulsion-type meat analogue products such as plant-based sausages are formed in a container such as casings, tin cans and jars. Casings can be made from natural materials (collagen, cellulose) or synthetic materials (plastic). Plant-based sausages are made in a wide range of varieties including frankfurters (e.g. wieners, hot dogs) and cold cuts (e.g. ham, pepperoni, salami, bierwurst, bologna, mortadella). Frankfurters are examples of small diameter emulsion-type sausages which are consumed in both cold and hot conditions. Cold cuts products such as ham, pepperoni, bierwurst, bologna, mortadella, are made in larger diameters and generally sliced for cold consumption. These products generally consist of substantial amount of water, plant-based proteins, vegetable oils, texturizing ingredients, salt and spices.

In animal-based emulsion-type products, soluble myofibrillar proteins derived from finely chopped meat in water and salt have water-holding ability, gelling and emulsifying properties. These properties contribute to the desired textural characteristic of the product. Plant-based proteins such as soy and pea proteins commonly used in plant-based foods are inferior to myofibrillar proteins in terms of functionalities in emulsion-type products, especially solubility and gelling property. The presence of plant-based proteins often leads to a reduction in gel formation/elasticity and undesirable textural attributes in of cooked emulsion-type meat analogues. Therefore, several texturizing ingredients are used to compensate the absence of meat protein functionalities. In vegetarian meat analogues, egg white is commonly added to provide the desired gelling property which improve product consistency. For vegan emulsion-type products, several food additives such as methylcellulose, carrageenan, konjac gum, locust bean gum and alginate are used as texturizing ingredients to improve textural properties for hot and cold consumptions. Cold and hot stabilities of the product are required for optimal sliceability, hardness, juiciness, and bite characteristics. For cold-used products, the cold stability and gel strength is the most important.

Achieving desirable cold and hot stabilities for emulsion-type vegan meat analogues remain a key challenge for food producers. A mixture of texturizing ingredients is used to optimize viscosity for processing and to improve cold and hot stabilities of the products. Viscosity of emulsion can be increased by cold thickening agents such as psyllium husk, pre-gelatinized starch, konjac, or oat fiber. The cold stability commonly relies on cold-set gelling agents such as carrageenan, konjac, agar-agar, alginate, guar gum, locust bean gum or starch. Carrageenan is the most used cold-set gelling agent, generally in combination with konjac and/or potassium chloride. The systems of carrageenan-konjac, carrageenan-potassium chloride and carrageenan-konjac-potassium chloride form synergistic composite gels at low temperature, exhibiting greater gel strength and cohesive behavior. These gels, however, melt upon increased temperature. Hot-set gelling agent such as methylcellulose, in contrast, forms gel upon heating. Its gel is thermo-reversible and melt upon cooling.

WO 2021/110760, for example, describes a sausage analogue. According to this publication, potato protein, kappa-carrageenan, konjac gum, potassium chloride are included in the composition to achieve desired emulsion properties. Carrageenan and konjac gum are present in a ratio of 3:2, while the ratio of carrageenan and konjac gum to potassium chloride is 9:1.

WO2015153666, for example, relates to ground plant-based products that mimic ground meat, including the fibrousness, heterogenicity in texture, beefy flavour, and red-to-brown colour transition during cooking of ground meat. EP3125699, for example, describes a meat replica composition comprising an isolated plant protein, an edible fibrous component, a carbohydrate-based gel, non-animal fat, cucumber or melon extract, a binding agent and a heme-containing protein and/or iron salt.

Current state of the art texturizing systems for cold and hot stabilities applied in plant-based emulsion-type meat analogues are composed of kappa-carrageenan and konjac gum (generally with additional potassium chloride) combined with methylcellulose. Synergistic gels from kappa-carrageenan and konjac contribute to viscosity during cold processing and desired characteristics for cold consumption such as high consistency, firmness/hardness, springiness of the products. Methylcellulose provides consistency, firmness and textures for hot consumption. Consequently, a mixture of several ingredients is required to create textural properties equivalent to that of animal-derived proteins such as egg white and myofibrillar proteins.

Despite the commercial application of these texturizing ingredients in vegan meat analogues, a long ingredient list of food additives with unfamiliar scientific equivalent names, such as konjac gum, konjac glucomannan, methylcellulose, on the package does not appeal to consumers. Therefore, there is a high demand for plant-based meat analogues containing ingredients that are natural, label-friendly, and more nutritional.

Konjac gum and konjac glucomannan are two water-soluble hydrocolloids that can be used as a thickener and cold gelling agent with the European food additive number E425(i) and E425(ii), respectively. They are obtained from konjac flour. Konjac gum is obtained by aqueous extraction, while konjac glucomannan is obtained by washing with water-containing ethanol. Konjac flour is defined as the unpurified raw product from the root of the perennial plant *Amorphophallus konjac.* Its main component is a high-molecular-weight polysaccharide glucomannan which consists of d-mannose and d-glucose units at a molar ratio of 1.6:1.0, connected by β(1-4)-glycosidic bonds. The difference between these two ingredients is mainly the purity of glucomannan in them. This content is required ≥ 75% in konjac gum, while needed more than 95% in konjac glucomannan, on a dry weight basis. According to Commission Regulation (EU) No 231/2012, the average molecular weight of the polysaccharide in konjac gum is in the range of 200,000-2,000,000 and in konjac glucomannan 500,000-2,000,000. One disadvantage with konjac gum or glucomannan is that it is difficult to incorporate into a number of food products. Without being bound by theory, it is considered that this is due to the hydrophilic nature of glucomannan, which often causes it to take on water from other ingredients present in the food product. If too much water is absorbed by the konjac glucomannan prior to consumption of the food product, then the konjac will not adequately swell in the consumer's stomach. It is therefore an advantage that the present invention does not require konjac gum or konjac glucomannan to achieve good cold stability of the vegan meat analogue.

The problem underlying the present invention is to provide a composition that improves the texture of plant-based emulsion-type meat analogues, such as vegan sausages, both for hot and cold conditions, or for cold conditions. Texture aspects relevant for consumer acceptance are high firmness, juiciness, springiness, chewiness and a bite experience on parity with traditional meat-based products.

The present invention provides a composition with minimal ingredients and a simplified manufacturing process to produce the emulsion-type meat analogues with desirable texture for cold and preferably also hot consumption. Particularly, the invention provides a meat analogue in a casing, wherein the meat analogue is an emulsion-type emulsion, with good cold and preferably hot stability.

The invention furthermore provides a dry composition, suitable to make the inventive meat analogue products.

Further benefits of the described dry composition and the meat analogues obtainable with it, are that food additives such as konjac, methylcellulose, or non-vegan ingredients such as egg white can be replaced, allowing products with shorter ingredient list and to be labeled vegan instead of vegetarian and thereby addressing a larger consumer group.

### SUMMARY OF THE INVENTION

The present invention provides a composition for manufacturing an improved emulsion-type meat analogue product. The composition comprises plant-based protein rubisco and kappa-carrageenan in relative amounts between 1:5 to 30:1.

The products comprising the invention possess desired textures at cold and preferably also hot conditions without the need (or with substantially less need) of conventional texturizing agents such as konjac gum or konjac glucomannan, methylcellulose or both. The products comprising the invention have excellent cold stability and can be sliced to desired thickness using cutting machine normally employed for meat cold cuts.

Rubisco is a protein ingredient having high solubility and gelling properties. WO2021/007484, for example, describes a production process of leaf protein (Rubisco) from lemna, an aquatic plant. Rubisco is a soluble protein having high hot-set gelling property at low concentration which is an uncommon characteristic for plant-based proteins.

Carrageenan has been described to have electrostatic interactions with some soluble animal-derived proteins such as gelatin, myosin, egg white and milk proteins. The synergistic interaction results in increased gel strength of the system. A publication by Lomolino (Lomolino et al. Food *Chemistry: X* 13 (2022) 100232) describes the emulsifying activity of potato proteins in the presence of kappa-carrageenan at low pH conditions. Properties like emulsion creaming and particle droplet size were improved by a combination of potato protein and kappa-carrageenan at very low pH 3. In contrast, minimal effects and decreased emulsion stability were observed at pH 7. Requirement of low pH condition limits the applications in emulsion-type meat analogues, as meat analogues generally require an around neutral pH.

The combination of rubisco and kappa-carrageenan appears to provide synergistic gelling properties, particular suitable for plant-based emulsion-type sausages that are generally provided to consumers in a container or casing such as frankfurters (e.g. wiener, hot dogs) or cold cuts (e.g. ham, pepperoni, salami, bierwurst, bologna, mortadella). The combination of rubisco and kappa-carrageenan can remove the need for konjac gum or konjac galactomannan, but may also reduce or eliminate the use of methylcellulose, providing the products with improved textures for cold and/or hot consumption. Accordingly, the improved emulsion-type meat analogues can be produced with fewer ingredients.

Preferably, the amounts of rubisco and carrageenan are selected such that it leads to a synergistic action in enhancing gel texture which is sufficient to achieve the desirable texture of the meat analogue products without (or substantially reducing) the use of some conventional texturizing agents such as konjac gum or konjac glucomannan and/or methylcellulose.

Generally, the amounts of rubisco and carrageenan are 0.5-30 wt%- and 0.1-15 wt% in the finished meat analogue product.

The pH of the emulsions generally is between 4-9, preferably between 5-8.

The inventive composition can be provided as a kit of parts comprising the necessary ingredients; as a dry composition that comprises the essential ingredients according to the present invention; or as a meat analogue product.

### DETAILED DESCRIPTION OF THE INVENTION

The present invention is based on a composition described herein which surprisingly delivers exceptional good quality of the emulsion-type meat analogue products with fewer ingredients in comparison to the prior art. The composition provides a unique synergistic interaction between the high gelling, soluble plant-based protein rubisco and kappa-carrageenan, providing a food system with high gel strength without the needs of konjac and potassium chloride, commonly used in combination with kappa-carrageenan in prior art. The composition may also reduce or eliminate the use of methylcellulose, providing the products with improved textures for cold and/or hot consumption. Accordingly, the improved emulsion-type meat analogues can be produced with fewer ingredients.

The composition preferably also comprises other ingredients that contribute to overall desired cold and hot stabilities of the products.

In a preferred embodiment, the composition comprises:
a. high gelling, soluble plant-based protein comprising rubisco;
b. carrageenan comprising kappa-carrageenan;
c. vegan protein other than rubisco;
d. texturizing ingredients other than rubisco and carrageenan

The composition system according to the invention allows to create an emulsion dough with sufficient viscosity for case filling, thereby allowing to produce plant-based meat analogues in a casing such as wieners, hot dogs, ham, pepperoni, salami, bierwurst, bologna, mortadella at exceptional textural properties at cold and hot consumption, while removing unwanted food additives such as konjac gum or konjac galactomannan, and/or methylcellulose.

Surprisingly, the vegan emulsion-type products produced by the new composition provides desired firmness and juiciness. One major advantage resulting therefrom is reduction of total number of ingredients by replacing potato protein and/or other plant proteins, konjac gum or konjac galactomannan, potassium chloride, and/or methylcellulose with the unique synergistic effects between kappa-carrageenan and rubisco protein.

Therefore, in a preferred embodiment of the present invention, the meat analogue does not comprise konjac gum or konjac galactomannan. In a further preferred embodiment, the meat analogue does not comprise methylcellulose. It is most preferred that none of these components is present.

The product according to the invention can be allergen-free as it does not need allergic ingredients like soy, wheat, egg, or milk constituents. Hence, in one embodiment of the invention, the meat analogue does not comprise constituents that are commonly known to be allergic, such as soy, wheat, egg, or milk constituents.

(a) High gelling high soluble plant-based protein comprising rubisco: Rubisco (ribulose-1,5-bisphosphate carboxylase-oxygenase) is one of the most abundant plant proteins on the earth. It is the enzyme involved in the photosynthetic fixation of carbon dioxide. This protein is water soluble and can be isolated from various plants and algae, and procedures for its isolation and purification have been disclosed, for instance in WO 2021/007484 and WO 2021/015087.

Preferably, rubisco protein isolate is obtained from *Lemna* or *Lemnoideae,* commonly known as duckweed, water lentils or water lenses. Alternatively, rubisco can be produced from other plant leaf materials like spinach leaf, grass leaf, sugar beet leaf, alfalfa leaf, or tobacco leaf. Rubisco is water soluble and possess a unique heat-induced gelation at low concentration which is different from most plant proteins. In addition, rubisco protein is a sustainable source of essential amino acids similar to those of egg white and soy proteins, and superior to proteins from pea, wheat and many plant sources. The high solubility of rubisco provides additional advantages in mixing, processing, accessibility for interaction with other ingredients and homogeneity of the products.

Other proteins are known to be moderately soluble, such as potato proteins and canola proteins, which may be used in combination with rubisco protein. Potato proteins can be categorized into three major groups, including patatin, protease inhibitors and high molecular weight (MW) proteins with relative contents of ~40%, ~50% and ~10%, respectively. It has been well documented that for example patatin possesses good functional properties, including moderate solubility in water. Canola/rapeseed protein is a co-product of oil extraction. The main protein fractions include cruciferin (11S globulin) and napin (2S albumin). Canola protein obtained from mild extraction process has moderate solubility in water.

Both potato patatin and canola proteins can form heat-induced gel at higher concentration than rubisco. Hence, potato patatin and canola protein may be used in combination with rubisco in compositions according to the present invention.

Patatin is a storage potato tuber protein that has lipase activity. It causes hydrolysis of phospholipids, monoglycerides and triglycerides with short chain fatty acids. Coconut oil and palm kernel oil, commonly used in a preparation of meat analogues, contain high amount of short chain fatty acids than other vegetable oils. Hydrolysis of these oils by potato patatin may generate a high amount of free fatty acids which may develop off-flavour in meat analogue products. In this regard, rubisco protein is preferred to potato patatin.

Other high gelling and soluble proteins that can be used in combination with rubisco may be derived through manufacturing methods such as mild extraction of plant proteins, extraction of soluble proteins from single cells, such as microalgae, yeast, bacteria, and proteins derived from fermentation process using genetic engineered microorganisms.

In its pure, commercially available form, rubisco protein, potato protein, canola protein or other gelling proteins are generally available as colorless or brownish or yellowish or off-white and neutral taste powder. Upon heating, the viscosity of the gelling, soluble proteins increase resulting in irreversible gel structures, while methylcellulose gel is thermo-reversible and melts upon cooling. Therefore, such proteins contribute to both hot and cold stabilities of the product, unlike methylcellulose.

The rubisco protein may be provided as soluble protein product, preferably containing about 50 wt% or more native rubisco protein, more preferably about 70%, and even more preferably about 90 wt% or more.

The high gelling, soluble plant-based protein comprising rubisco is generally used in an amount between 0.5% and 30%, wherein the % is indicated as wt% related to the plant-based meat analogues. In a preferred embodiment of the present invention, the amount of rubisco is 1.0 - 15%.

(b) Carrageenan: Carrageenan is a generic name for a family of gel-forming and viscosifying polysaccharides, which are obtained by extraction from certain species of red seaweeds. Carrageenan is extracted from red seaweed of the Rhodophyceae family. This particular type of seaweed is common in the Atlantic Ocean near Britain, Europe and North America. When used in food products, carrageenan has the EU additive E-number E407 or E407a, also known as processed Eucheuma seaweed, which corresponds to semi-refined carrageenan. E407a has a slightly different composition; moreover, it contains a considerable amount of insoluble materials.

Carrageenan is a sulfated polygalactan with an average relative molecular mass well above 100 kDa and 15 to 40% of ester-sulfate content, which makes it an anionic polysaccharide. Carrageenans are linear sulfated polysaccharides formed by alternate units of D-galactose and 3,6-anhydro-galactose (3,6-AG) joined by α-1,3 and β-1,4-glycosidic linkage. The number and the position of ester sulfate groups and the content of 3,6-AG influences in the chemical reaction property of carrageenan. Based on the solubility rate in potassium chloride, carrageenan is classified into λ, κ, ι, ε, µ carrageenan where all contains 22-35% of sulfate groups. These names do not reflect definitive chemical structures but only general differences in the composition and degree of sulfation at specific locations in the polymer. Kappa-carrageenan (κ) contains 25-30% of ester sulfate and 28-35% of 3,6- AG contents; Iota-carrageenan (ι) contains 28-30% of ester sulfate and 25-30% of 3,6-AG contents; Lambda carrageenan (λ) contains 32-39% of ester sulfate and no 3,6-AG contents. The level of ester sulfate is indirectly proportional to the gel strength, solubility, and temperature stability. Hence, higher levels of ester sulfate mean lower solubility temperature and lower gel strength. The increased ester sulfate level lowers the mechanical property of sulfated polysaccharides. The pH conditions influence the carrageenan activity, wherein low pH disturbs its action. Kappa-2 carrageenan is a copolymers containing kappa and iota repeating units covalently bound in clear distinction to physical mixtures of kappa and iota carrageenan. This hybrid type is derived from cold water seaweeds such as *Chondrus Crispus*, *Sarcothalia crispate*, *Gigartina skottsbergii.*

The chemical reactivity of carrageenan is primarily due to their half-ester sulfate groups which are strongly anionic, being comparable to inorganic sulfate in this respect. The free acid is unstable, and commercial carrageenan products are available as stable sodium, potassium and/or calcium salts or, most commonly, as a mixture of these. The associated cations together with the conformation of the sugar units in the polymer chain determine the physical properties of the carrageenans. *(*Necas, J., Bartosikova, L. (2013) Carrageenan: a review. Veterinarni Medicina, 58 (4): 187-205*).*

The composition according to the present invention comprises kappa-carrageenan in an amount between 0.1% and 15%, preferably between 0.5% and 5% , wherein the % is indicated as wt% are related to the plant-based meat analogues. In a preferred embodiment of the present invention, the carrageenan is kappa-carrageenan, but mixtures of kappa-carrageenan with other carrageenan's can be used as well.

The composition according to the present invention comprises rubisco plant-based protein and kappa-carrageenan, in relative amounts between 1:5 to 30:1. Preferably, the relative amounts are 1 part of carrageenan to 1-10 parts of (1:1 to 1:10) rubisco protein, more preferable 1 to 1-5 (1:1 to 1:5).

(c) Vegan protein: In a preferred embodiment of the present invention, a vegan protein component (other than rubisco) is used in the composition according to the present invention. Vegan protein means any protein derived from plants or a microbial protein material or a mixture thereof, which is suitable for nutrition purposes, in particular for human nutrition. In particular, the protein material does not contain any protein of animal origin.

This vegan protein (also called plant-based protein) other than rubisco generally contributes to water and oil holding capacity, mass viscosity and total protein content of the product, and can be considered 'bulk protein'.

One type of vegan proteins is potato and/or canola proteins, discussed above, having moderate solubility.

The composition according to the present invention preferably comprises a vegan protein other than rubisco, selected from the group of protein isolates or concentrates. These protein isolates or concentrates generally have relatively poor solubility. Examples of vegetable proteins suitable for use in such composition are protein materials from pulses, such as chickpea, faba bean, lentils, lupine, mung bean, pea or soy, protein materials from oil seed, such as hemp, rapeseed/canola or sunflower, protein materials from cereals, such as rice, corn, wheat or triticale, further potato protein, protein materials from nuts, such as almond, cashew, peanut, protein materials from plant leaves such as alfalfa leaves, spinach leaves, sugar beet leaves or water lentil leaves, microbial proteins, algae proteins and/or mixtures thereof. Examples of microbial proteins, which are also termed single cell proteins (SCP) include proteins from fungi, proteins from mycelium, proteins from archaea, proteins from bacteria, proteins from yeast, and proteins from microalgae.

In a preferred embodiment, the plant-based protein other than rubisco is obtained from faba bean, pea, soybean, wheat, canola (rapeseed), chickpea, lupin, almond, mung bean, single cells (microalgae, yeast, bacteria, archaea) or combinations thereof. Even more preferably, protein isolates and/or concentrates of soy, pea, faba bean and/or canola are used.

Pea protein includes four major classes (globulin, albumin, prolamin, and glutelin), in which globulin and albumin are major storage proteins in pea seeds. Pea protein has a well-balanced amino acid profile with high level of lysine. The composition and structure of pea protein, as well as the processing conditions, significantly affect its physical and chemical properties, such as hydration, rheological characteristics, and surface characteristics. Pea protein ingredients can be obtained through wet extraction, dry fractionation or more recently mild fractionation. As such, commercial pea proteins ingredients include flour (20-25% protein), concentrate (50-75% protein), and isolate (>80% protein). Faba beans contain almost twice the protein content as that in cereal grains with globulins (60%), albumins (20%), glutelins (15%), and prolamins (8%). Faba bean possesses high protein content from 20% to 41% wherein the wide variations are due to varietal differences and the source type, that is, flour, fraction, or isolate, as well as fertilization method, growth season, and planting site.

The vegan protein other than rubisco is preferably used in an amount of 0.5 -30%, more preferably 1 - 20%, wherein the % is wt% related to the plant-based meat analogues.

d. Texturizing ingredients: Texturizing ingredients are added to optimize emulsion viscosity, and textural properties at cold and optionally hot conditions.

Generally, the texturizing ingredients are present in an amount of 0.1 - 30 wt% related to the plant based meat analogue.

In a preferred embodiment of the present invention, the texturizing ingredients comprise a cold-thickening agent. The cold thickening agent in the plant-based meat analogue is a polysaccharide that is completely or partially soluble in water at room temperature (20-30°C) with or without shearing process, and their water mixtures possess high viscosity without heat pretreatment. The cold thickening agent provides cold adhesiveness and viscosity of emulsion at room temperature, which is useful to optimize the viscosity of emulsion dough for stuffing and filling to a container or casing in a production of plant-based emulsion-type sausages such as frankfurters (e.g. wiener, hot dogs), cold cuts (e.g. ham, pepperoni, salami, bierwurst, bologna, mortadella).

The cold-thickening agent is preferably psyllium husk, pre-gelatinized starch or their mixtures. In an embodiment of the present invention, psyllium husk is particularly preferred. Other suitable texturizing ingredients include starches, oat fiber, wheat fiber, citrus fiber, maltodextrins with low dextrose equivalent, and partially or completely soluble fibers or gums, such as beta-glucan, arabinoxylan, xyloglucan, pectin, xanthan gum, guar gum, konjac glucomannan, gum tragacanth, carrageenan, gellan gum, scleroglucan, locust bean gum, alginate. Konjac gum and konjac glucomannan preferably are not used.

In one preferred embodiment of the present invention, the texturizing agent is methylcellulose. Methylcellulose preferably is used further improve hot stability of the products such as in frankfurters (hot dog). When used, methylcellulose is present in an amount of 0.1 - 15%, preferably 0.1 - 4.0%, wherein the % is wt% related to the plant-based meat analogues. For, for example, cold cuts that are not served warm, in a preferred embodiment of the present invention, methylcellulose is not used.

Methylcellulose (MC) is one of the most important commercial cellulose ethers and it has been used in many industrial applications. Methylcellulose does not occur naturally and is synthetically produced by heating cellulose with caustic solution (e.g. sodium hydroxide) and treating it with methyl chloride. MC is the simplest cellulose derivative, where methyl groups (-CH3) analogue the hydroxyls at C-2, C-3 and/or C-6 positions of anhydro-D-glucose units. This cellulose derivative has amphiphilic properties and original physicochemical properties. MC becomes water soluble or organo-soluble when the degree of substitution (DS) is below 3.

The addition of methyl groups makes the cellulose molecules more hydrophobic, but it also allows water molecules to penetrate between the cellulose chains and disrupt the hydrogen bonds that normally hold them together (which makes natural cellulose insoluble in water). Methylcellulose forms a reversible gel upon heating above 52 °C, which is due to the increase in the strength of the hydrophobic attractive forces between the methyl groups. It has both nonpolar (methyl) and polar (hydroxyl) groups on its surfaces, which means that it can interact with both hydrophobic and hydrophilic substances within its environment, thereby contributing to its binding properties.

*Other components*: in addition to components a-d described above, the meat analogue composition may comprise further ingredients. The other components may be used in amounts between 0.01 wt% to 30 wt% or more. Preferably, the composition according to the present invention comprises one or more of the following ingredients:
- Flavoring agents: some plant proteins used in the manufacturing of meat analogues may have unpleasant flavor profiles and intrinsic off-flavors, which hinders the acceptability of the products. Further, apart from correcting the off-flavors, desired aroma and taste needs to be introduced depending on the final product to be obtained. Flavoring agents suitable for use in compositions according to the present invention may be produced using precursors like reducing sugars (glucose, xylose, fructose, and ribose), amino acids (cysteine, cystine, lysine, methionine, proline, serine, threonine), vitamins (such as thiamine), nucleotides and iron complexes (e.g., ferrous chlorophyllin or heme-containing proteins). Further, flavoring agents can also be selected from the group consisting of a vegetable extracts and/or vegetable powders (e.g. onion powder, garlic powder, tomato powder or celery root powder), herbs and/or spices (parsley, celery, fenugreek, lovage, rosemary, marjoram, dill, tarragon, coriander, ginger, lemongrass, curcuma, chili, ginger, paprika, mustard, garlic, onion, turmeric, tomato, coconut milk, cheese, oregano, thyme, basil, chillies, paprika, pimenton, jalapeno pepper, white pepper powder or black pepper), fruit extracts, antioxidants (e.g. epigallocatechin gallate), carotenoids (e.g. lutein, β-carotene, zeaxanthin, trans-β-apo-8'-carotenal, lycopene, or canthaxanthin), lactones, and/or combinations thereof. Together with aromas and precursors, salt also plays an important role in taste perception. However, the role of salt is not only as a taste enhancer, but it also contributes to the extension of the shelf life of the product. Therefore, the composition according to the present invention may contain salts as sodium chloride or potassium chloride. Flavoring ingredients generally are used in amounts from 0.01 to 10%, preferably 0.5 to 10 wt%, preferably 0.5 to 7 wt%, preferably 0.5 to 5 wt%, preferably 1 to 10 wt%, preferably 1 to 5 wt% wherein % is wt% are related to the plant-based meat analogues.
- Coloring agents: most commonly used ingredients for meat analogues have a beige or yellow-brown color. Thus, the color of the basic meat analogue structure is far different from the well-accepted red color of unprocessed meat or the brown color of cooked meat. Therefore, coloring agents may be added to the composition according to the present invention. The coloring compositions of the invention may comprise a thermally unstable pigment, a thermally stable pigment, and/or a browning agent. The choice of the type of coloring agents and the amount present in the composition may vary depending upon the desired color of the final meat analogue. Coloring agents suitable for use in compositions according to the present invention can be selected from the groups consisting of artificial dyes (e.g. FD&C Yellow #5, yellow #6, azo dye, triphenylmethane, xanthene, quinine, indigoid, titanium dioxide, red #3, red #40, blue #1,), cumin, saffron, carotenoids (e.g. carotene, canthaxanthin and lycopene), carotenoid-rich extracts (e.g. paprika oleoresins), caramel colors, malt, annatto, turmeric, beet pigment, iron oxide, anthocyanin, reducing sugars, amino acid sources, erythrosine, red yeast (*Monascus purpureus*) products made by fermenting rice and/or combinations thereof. The composition according to the present invention may further comprise an acidity regulator to maintain the pH in the optimal range for the colorant. The acidity regulator may be an acidulent. Examples of acidulants that may be added to the composition include citric acid, acetic acid (vinegar), tartaric acid, malic acid, fumaric acid, lactic acid, phosphoric acid, sorbic acid, and benzoic acid. Coloring ingredients may be used in amounts between 0.005 and 10 wt%, wherein % is wt% are related to the plant-based meat analogues. whereby higher amounts are used when substances are used with relatively low coloring power, like beet extracts.
- Vitamins, minerals and iron salts: the composition according to the present invention may include vitamins, such as vitamin A, vitamin B-complex (e.g. B-1, B- 2, B-6 and B-12), vitamin C, vitamin D, vitamin E and vitamin K, niacin, acid vitamins such as pantothenic acid, folic acid, biotin, minerals such as calcium, iron, zinc, magnesium, iodine, copper, phosphorus, manganese, potassium, chromium, molybdenum, selenium, nickel, tin, silicon, vanadium, iron salts such as ferric sodium EDTA, reduced iron, ferrous lactate, ferric citrate, ferric pyrophosphate, ferrous sulphate monohydrate or ferric ammonium citrate brown, and/or combinations thereof. Vitamins, minerals and iron salts may be used in amounts less than 5 wt%, preferably less than 3 wt%, even more preferably less than 2 wt%, wherein % is wt% are related to the plant-based meat analogues.
- Structuring agents: incorporated into the composition, structuring agents which enhance the texture of the product to make it for consistent with that of a meat product, for example a meat sausage, may be used. The textured components are used to resemble a desired size of meat particles, for example, providing particles in the sausage. In order to mimic other types of sausages like e.g. coarse bratwurst it is also possible to incorporate hydrated and shredded textured vegetable protein (TVP) particles to resemble chunks of meat. Structuring agents are used in varying amounts depending on the type of structuring agent, and type of meat analogue, for example the type of sausage analogue, aimed for. For example, salami type and mortadella type may comprise 5-20 wt% fat-like particles. Generally, structuring agents are used in amounts of less than 70 wt%, wherein % is wt% are related to the plant-based meat analogues, preferably less than 60 wt%, more preferably less than 50 wt%, even more preferably less than 40 wt%. In a preferred embodiment, structuring agents. are used in amounts between 1-30 wt%,
- Fat replacers: vegan fat replacers may be selected from the group consisting of soybean oil, corn oil, sunflower oil, high oleic sunflower oil, olive oil, canola oil, safflower oil, peanut oil, palm oil, cottonseed oil, coconut oil, almond oil, hazelnut oil, rape seed oil, fractionated palm fat, fully or partially hydrogenated or inter-esterified palm oil and/or combinations thereof. Vegan fat replacers may also be produced by combinations of water, oil and starch. Tapioca starch particles may be used as fat replacer. Alternatively, such a system might also comprise alginate and/or methylcellulose. It may also be composed of other hydrocolloids that guarantee stability of contour of the individual particles within the sausage dough.

The ingredients listed above can be supplied as dry mixture, or kits of parts with dry constituents for providing the mixture.

As dry mixture, the amounts of in the dry mixture preferably are as follows:
a. high gelling, soluble plant-based protein comprising rubisco in amounts between 10 to 80 wt%, preferably between 15 to 75 wt%, more preferably between 20 to 70 wt%, even more preferably between 25 to 65 wt%, even more preferably between 30 to 60 wt%.
b. carrageenan comprising kappa-carrageenan in amounts between 1 to 40 wt%, preferably between 2 to 35 wt%, more preferably between 4 to 30 wt%, even more preferably between 6 to 25 wt%.
c. plant-based protein other than rubisco in amounts between 1 to 35 wt%, preferably between 5 to 30 wt%, more preferably between 7 to 28 wt%.
d. texturizing ingredients other rubisco and carrageenan in amounts between 1 to 35 wt%, preferably 5 to 30 wt%, more preferably between 7 to 28 wt%.

Although, preferably the components are supplied as dry mixture, it is possible to supply the components as kits of part, wherein for example the protein component is provided as one part, and the other components as a separate part, yet, with instruction to combine the two parts.

The dry composition can be mixed with water and oil to provide an emulsion as further described below.

The composition defined above generally is used in amounts between 2-40 wt%, preferably 5-30 wt%, with 30-90 wt% water and 2-60 wt% plant-based lipid, preferably 2-50 wt%, more preferably 2-40 wt%, even more preferably 5-30 wt% plant-based lipid.

Plant based lipid generally is an edible fat or oil. The lipid generally is a triglyceride oil having fatty acid chains of between 12-24 carbon atoms. Generally, at least 90% of the fatty acid chains have 14-20 carbon atoms. Suitable oils have a melting point of between -25 °C and 35 °C. Oils with higher degree of unsaturation have lower melting points, whereas for example cocoa butter melts at 34 °C. It is preferred to use oils with a melting point of about -5 to 35 °C, although this may also depend on the specific recipe. Suitable vegetable oils include corn, palm, rapeseed, canola, sunflower, soybean or coconut oil, and mixtures thereof. In a preferred embodiment, the oil comprises canola oil, rapeseed oil, sunflower oil, palm oil or coconut oil.

The present invention also relates to the use of the described composition to produce a plant-based food product.

By plant-based food product it is meant a product which is a meat analogue, i.e., devoid of meat protein. Beyond that, the plant-based food product here will mimic taste, texture, and appearance of a similar meat-based product, offering the consumer a viable alternative to cut their meat intake and still deliver on gustatory satisfaction.

In a preferred embodiment of the invention, the plant-based food product containing the composition is a sausage analogue. The term "sausage" is directed to an edible product (food product) which requires processing into an emulsion, followed by shaping and heating to solidify the product to a stable physical form. In this emulsion system the continuous phase is mainly composed of water and protein and further additives including those of the present invention and the dispersed phase is fat or oil which is embedded in the matrix and stabilized by protein or other materials with surface activity. The matrix ties the fat and water tightly into the whole system. As a result, the sausage performance is mainly determined by the matrix (continuous phase). Although the term "sausage" generally relates to a food product having an approximately cylindrical shape, similar products having the shape of a non-specific loaf are also encompassed herein by the term "sausage".

An emulsion-type sausage usually refers to a sausage with homogeneous consistency. In order to mimic special types of sausages like, e.g. coarse bratwurst, pepperoni or salami-style sausages, mortadella, it might be necessary to incorporate coarse particles, e.g. hydrated texturized vegetable protein (TVP) or large solid fat particles to create a heterogenous mass.

Meat analogue according to invention preferably does not comprise konjac gum or konjac glucomannan and has the following product characteristics:
- gel hardness at 15 °C of about 3000 g or higher
- gel hardness at 72 °C of about 2000 g or higher.

In a further embodiment, the meat analogue according to invention preferably does not comprise konjac gum or konjac glucomannan, and methylcellulose and has the following product characteristics:
- gel hardness at 15 °C of about 3000 g or higher
- gel hardness at 72 °C of about 900 g or higher.

Furthermore, the present invention also refers to the method of production of the described food product, wherein the method comprises the steps of:
- providing the composition according the invention;
- add a fat, like for example rapeseed oil, and generally some salt like sodium chloride;
- optionally add seasoning agent
- and emulsifying the mixture with water, preferably water at a temperature between 0-10 °C, more preferably between 0-5 °C, such as ice water.

In the method of production of the food product, as described above, the composition object of the invention is blended with ice water preferably in an amount of 55-80% by total weight. Rapeseed oil (or another lipid) is added preferably in an amount of 7-19% by total weight and sodium chloride in an amount of 0-2% by total weight. Seasonings are added optionally by discretion.

### EXAMPLES

The following examples are included to demonstrate preferred embodiments of the invention. The examples furthermore illustrate the synergistic effect of the preferred ingredient combination in comparison to other obvious combinations that do not exhibit the same synergy as the preferred embodiment which has been surprisingly discovered by the inventors.

### Viscosity of emulsion

The viscosity of the emulsions was determined using RVA4500 and TCW software (Perten Instrument). The test configuration followed the normal instructions: 30 g emulsion was weighed into an RVA canister, stirring speed was 160 rpm for 20 minutes at 15°C. The final viscosity was recorded. The measurements were carried out in duplicate.

### Hardness and gel strength

The term "hardness" as used herein refers to a texture parameter of a food and is calculated from the end point of the first compression of the food in a compression assay employing suitable measuring devices, as e.g. a Texture Analyzer or a similar.

The hardness (gel strength) of the protein gels and protein-hydrocolloid/fiber gels were assessed with a Texture Analyzer (TA.XT. plus, Stable Micro Systems). Hardness value is used to determine gel strength of the gel. The test mode was according to normal instructions, in compression mode; test speed 0.25 mm/s; distance 5 mm, trigger force was 5 g on a 10 ml gel sample using a cylindrical probe (0.5" diameter, Delrin^{®}, part code P/0,5).

The hardness (gel strength) of the sausages was assessed with a Texture Analyzer (TA.XT. plus, Stable Micro Systems). For the measurements sausages were sliced in pieces of 1.5 cm thickness and measured cold and hot at core temperatures of 15°C and 72°C. Hardness value is used to determine gel strength of sausage. The test mode was according to normal instructions, in compression mode; test speed 0.50 mm/s; distance 5 mm, trigger force was 5 g on the 1.5 cm thick slices using a cylindrical probe (25 mm diameter, Cyl. Perspex, part code P/25P).

The detected force is believed to correlate with the force required to compress the food between molars during mastication. Variables used herein to adjust the hardness of a meat-analogue are focused on concentrations and ratios of binding materials employed.

### Experiments 1-16 Interaction of Rubisco with various texturizing ingredients derived from hydrocolloids and fibers

In order to elucidate if Rubisco protein shows a synergistic effect with hydrocolloids and fibers, a pre-set concentration of rubisco protein, namely 4% w/w was mixed with 1% w/w of a hydrocolloid or fiber. Experiments were carried out with addition of iota-carrageenan, kappa-carrageenan, lambda-carrageenan, gellan gum, konjac gum, locust bean gum, guar gum, xanthan, pectin, tamarind xyloglucan, methylcellulose, carboxy methylcellulose, citrus fiber and psyllium husk. Furthermore, hydrocolloids were tested for their gel strength in a concentration of 1% w/w without addition of protein.

This dry mixture or individual hydrocolloid or fiber was dissolved in distilled water at room temperature, stirred and heated to reach a temperature of 55°C on a hot plate with magnetic stirrer. For samples containing 1% w/w hydrocolloid or fiber, 0.3 g of the hydrocolloid or fiber were prepared with 29.7 g of distilled water. Samples with protein addition were composed of 1.2 g protein, 0.3 g hydrocolloid or fiber and 28.5 g distilled water. The prepared mixtures were filled into 3 glass vials containing 10 g of sample each and heat up to 90 °C for one hour in order to induce gelation. Samples were cooled down at room temperature for two hours and stored in the fridge at 4 °C for 16 hours.

The hardness of the gels was assessed with a Texture Analyzer (TA.XT. plus, Stable Micro Systems). Measurements were performed in triplicate. The test mode was according to normal instructions, in compression mode; test speed 0.25 mm/s; distance 5 mm, trigger force was 5 g on a 10 ml gel sample using a cylindrical probe (0.5" diameter, Delrin^{®}, part code P/0,5). Samples were measured at a temperature of 4°C.

### Composition of experiments for hydrocolloid/fiber assessment [% w/w] Exp. 1a-5b

| | Exp. 1a | Exp. 2a | Exp. 2b | Exp. 3a | Exp. 3b | Exp. 4a | Exp. 4b | Exp. 5a | Exp. 5b |
|---|---|---|---|---|---|---|---|---|---|
| Rubisco protein | 4 | 0 | 4 | 0 | 4 | 0 | 4 | 0 | 4 |
| Kappa-Carrageenan | 0 | 1 | 1 | 0 | 0 | 0 | 0 | 0 | 0 |
| Kappa-2-Carrageenan | 0 | 0 | 0 | 1 | 1 | 0 | 0 | 0 | 0 |
| lota-Carrageenan | 0 | 0 | 0 | 0 | 0 | 1 | 1 | 0 | 0 |
| Lambda Carrageenan | 0 | 0 | 0 | 0 | 0 | 0 | 0 | 1 | 1 |
| pH | 6.96 | 10.03 | 7.05 | 10.04 | 7.55 | 10.34 | 8.02 | 9.11 | 7.04 |

### Texture assessment Exp. 1a-5b

| | Exp. 1a | Exp. 2a | Exp. 2b | Exp. 3a | Exp. 3b | Exp. 4a | Exp. 4b | Exp. 5a | Exp. 5b |
|---|---|---|---|---|---|---|---|---|---|
| Hardness [g] | 85 | 208 | 757 | 19 | 84 | 15 | 26 | 0 | 0 |

### Composition of experiments for hydrocolloid/fiber assessment [% w/w] Exp. 6a-11b

| | Exp. 6a | Exp. 6b | Exp. 7a | Exp. 7b | Exp. 8a | Exp. 8b | Exp. 9a | Exp. 9b | Exp. 10a | Exp. 10b | Exp. 11a | Exp. 11b |
|---|---|---|---|---|---|---|---|---|---|---|---|---|
| Rubisco protein | 0 | 4 | 0 | 4 | 0 | 4 | 0 | 4 | 0 | 4 | 0 | 4 |
| Gellan gum | 1 | 1 | 0 | 0 | 0 | 0 | 0 | 0 | 0 | 0 | 0 | 0 |
| Konjac gum | 0 | 0 | 1 | 1 | 0 | 0 | 0 | 0 | 0 | 0 | 0 | 0 |
| Locust bean gum | 0 | 0 | 0 | 0 | 1 | 1 | 0 | 0 | 0 | 0 | 0 | 0 |
| Guar gum | 0 | 0 | 0 | 0 | 0 | 0 | 1 | 1 | 0 | 0 | 0 | 0 |
| Xanthan | 0 | 0 | 0 | 0 | 0 | 0 | 0 | 0 | 1 | 1 | 0 | 0 |
| Pectin | 0 | 0 | 0 | 0 | 0 | 0 | 0 | 0 | 0 | 0 | 1 | 1 |
| pH | 4.94 | 6.75 | 5.80 | 6.92 | 5.61 | 6.81 | 5.91 | 6.83 | 6.85 | 6.94 | 4.17 | 5.41 |

### Texture assessment Exp. 6a-11b

| | Exp. 6a | Exp. 6b | Exp. 7a | Exp. 7b | Exp. 8a | Exp. 8b | Exp. 9a | Exp. 9b | Exp. 10a | Exp. 10b | Exp. 11a | Exp. 11b |
|---|---|---|---|---|---|---|---|---|---|---|---|---|
| Hardness [g] | 100 | 124 | 9 | 5 | 9 | 7 | 0 | 5 | 0 | 5 | 0 | 10 |

### Composition of experiments for hydrocolloid/fiber assessment [% w/w] Exp. 12a-16b

| | Exp. 12a | Exp. 12b | Exp. 13a | Exp. 13b | Exp. 14a | Exp. 14b | Exp. 15a | Exp. 15b | Exp. 16a | Exp. 16b |
|---|---|---|---|---|---|---|---|---|---|---|
| Rubisco protein | 0 | 4 | 0 | 4 | 0 | 4 | 0 | 4 | 0 | 4 |
| Tamarind xyloglucan | 1 | 1 | 0 | 0 | 0 | 0 | 0 | 0 | 0 | 0 |

| | | | | | | | | | | |
|---|---|---|---|---|---|---|---|---|---|---|
| Methylcellulose | 0 | 0 | 1 | 1 | 0 | 0 | 0 | 0 | 0 | 0 |
| CarboxymethylCellulose | 0 | 0 | 0 | 0 | 1 | 1 | 0 | 0 | 0 | 0 |
| Citrus fibre | 0 | 0 | 0 | 0 | 0 | 0 | 1 | 1 | 0 | 0 |
| Psyllium husk | 0 | 0 | 0 | 0 | 0 | 0 | 0 | 0 | 1 | 1 |
| pH | 7.10 | 6.87 | 6.78 | 6.94 | 6.46 | 6.89 | 4.49 | 6.07 | 5.34 | 6.92 |

### Texture assessment Exp. 12a-16b

| | Exp. 12a | Exp. 12b | Exp. 13a | Exp. 13b | Exp. 14a | Exp. 14b | Exp. 15a | Exp. 15b | Exp. 16a | Exp. 16b |
|---|---|---|---|---|---|---|---|---|---|---|
| Hardness [g] | 0 | 9 | 0 | 24 | 0 | 0 | 0 | 0 | 17 | 17 |

The preferred embodiment of Experiment 2b with a combination of 4% w/w rubisco protein and 1% w/w kappa-carrageenan revealed significantly higher gel hardness compared to gel hardness of the individual ingredients at equivalent concentration, indicating synergistic interaction in gel formation. Before heating, the mixtures of rubisco and kappa-carrageenan mixtures are nearly homogenous dispersion. After heating, the gel also showed homogenous appearance without particles or lumps. Furthermore, combinations of 4% rubisco protein and 1% w/w of carrageenan types other than kappa-carrageenan, namely kappa-2-carrageenan, iota carrageenan and lambda carrageenan did not show the same effect, and exhibited gel hardness inferior to the preferred embodiment of Experiment 2b.

Experiments carried out with addition of gellan gum, konjac gum, locust bean gum, guar gum, xanthan, pectin, tamarind xyloglucan, methylcellulose, carboxy methylcellulose, citrus fiber and psyllium husk, revealed low gel hardness or no gel formation at all. Under the given conditions, the results showed that except for kappa-carrageenan, other types of hydrocolloids and fibers would interfere with rubisco protein gel formation, resulting in a system without gel formation or very weak gel structures.

### Experiments 17-20 Comparison of different Rubisco protein concentrations

In order to elucidate if rubisco protein shows a synergistic effect at different protein levels with the same amount of kappa-carrageenan, respective amounts w/w were mixed with 1% w/w of kappa-carrageenan.

The dry mixtures or protein alone were dissolved in distilled water at room temperature, stirred and heated to reach a temperature of 55°C on a hot plate with magnetic stirrer. The prepared mixtures were filled into 3 glass vials containing 10g of sample each and heated up to 90°C for one hour in order to induce gelation. Samples were cooled down at room temperature for two hours and stored in the fridge at 4°C for 16 hours.

The hardness of the gels was assessed with a Texture Analyzer (TA.XT. plus, Stable Micro Systems). Measurements were performed in triplicate. The test mode was according to normal instructions, in compression mode; test speed 0.25 mm/s; distance 5 mm, trigger force was 5 g on a 10 ml gel sample using a cylindrical probe (0.5" diameter, Delrin^{®}, part code P/0,5). Samples were measured at a temperature of 4°C.

### Composition of experiments for different Rubisco protein concentrations [% w/w] Exp. 17-20

| | Exp. 17 | Exp. 18a | Exp. 18b | Exp. 19a | Exp. 19b | Exp. 20a | Exp. 20b |
|---|---|---|---|---|---|---|---|
| Rubisco protein | 0 | 2 | 2 | 3 | 3 | 5 | 5 |
| Kappa-Carrageenan | 1 | 0 | 1 | 0 | 1 | 0 | 1 |
| pH | 10.03 | 7.10 | 7.13 | 7.06 | 7.07 | 6.96 | 7.03 |

### Texture assessment Exp. 17-20

| | Exp. 17 | Exp. 18a | Exp. 18b | Exp. 19a | Exp. 19b | Exp. 20a | Exp. 20b |
|---|---|---|---|---|---|---|---|
| Hardness [g] | 208 | 21 | 361 | 47 | 457 | 138 | 580 |

The experiments demonstrate that synergy of Rubisco protein and kappa-carrageenan exists across a range of different protein concentration levels.

### Examples 1-3; comparative experiments A-B Comparative formulations for vegan cold cuts

Different formulations for vegan cold cuts were prepared according to the following process: The manufacturing of the sausage emulsion dough is done in a high-speed bowl chopper that can also apply vacuum. Oil and dry ingredients are added to the bowl chopper and mixed (2000 rpm). Then water/ice 1:1 is added. The mixture is blended for 3 min. to create the plant-based sausage dough. Sodium chloride and spices are added and blending continued for a few rounds. The dough is blended under reduced pressure or vacuum until a temperature of 10-12°C is reached. The created sausage dough is filled under reduced pressure into suitable casings of 80 mm diameter.

Sausages were cooked at 92°C for 1:45h to a core temperature of >85°C. Afterwards, sausages were cooled down in cold water to room temperature and stored in a fridge at 4°C for at least 24 hours.

The hardness (gel strength) of the sausages was assessed with a Texture Analyzer (TA.XT. plus, Stable Micro Systems). For the measurements sausages were sliced in pieces of 1.5 cm thickness and measured cold and hot at core temperatures of 15°C and 72°C. The test mode was according to normal instructions, in compression mode; test speed 0.50 mm/s; distance 5 mm, trigger force was 5 g on the 1.5 cm thick slice using a cylindrical probe (25 mm diameter, Cyl. Perspex, part code P/25P).

For stabilization of the system in comparative experiment A, a state-of-the-art formulation based on kappa-carrageenan, konjac gum and potato protein was prepared. In example 1 potato protein was replaced by rubisco protein. Comparative experiment B comprises Rubisco as sole texturizing component, Examples 2 and 3 reveal the synergy of kappa-carrageenan and rubisco, while the preferred composition of Example 3 shows comparable texture characteristics as the current state-of-the-art formulation A.

### Composition (% by weight) of vegan cold cut products

| Ingredients | Comp. Exp. A | Example 1 | Comp. Exp. B | Example 2 | Example 3 |
|---|---|---|---|---|---|
| Water/Ice | 73.45 | 73.45 | 76.75 | 75.75 | 74.25 |
| Rapeseed Oil | 12 | 12 | 12 | 12 | 12 |
| Methylcellulose | 1.25 | 1.25 | 1.25 | 1.25 | 1.25 |
| Psyllium husk | 2 | 2 | 2 | 2 | 2 |

| | | | | | |
|---|---|---|---|---|---|
| NaCl | 2 | 2 | 2 | 2 | 2 |
| Faba bean protein | 2 | 2 | 2 | 2 | 2 |
| Carrageenan | 2.5 | 2.5 | 0 | 1 | 2.5 |
| Konjac gum | 0.8 | 0.8 | 0 | 0 | 0 |
| Rubisco protein | 0 | 4 | 4 | 4 | 4 |
| Potato protein | 4 | 0 | 0 | 0 | 0 |

### pH and viscosity of emulsion before filling into casing

| | Comp. Exp. A | Example 1 | Comp. Exp. B | Example 2 | Example 3 |
|---|---|---|---|---|---|
| pH | 6.6 | 6.7 | 6.6 | 6.7 | 6.7 |
| Viscosity at 15°C (mPas) | 19046 | 16755 | 7463 | 8496 | 9769 |

### Texture assessment (Description and Hardness measurement)

| | Comp. Exp. A | Example 1 | Comp. Exp. B | Example 2 | Example 3 |
|---|---|---|---|---|---|
| Description of cold texture | Firm | Firm | Pasty | Pasty | Firm |
| Hardness cold (15°C) [g] | 3832 | 4563 | 443 | 1820 | 3836 |
| SD Hardness cold (15°C) | 126 | 83 | 37 | 217 | 121 |
| Hardness hot (72°C) [g] | 2310 | 2901 | 1104 | 999 | 2524 |
| SD Hardness hot (72°C) | 150 | 179 | 50 | 171 | 157 |

From example 3, it follows that konjac gum is not required to achieve good cold and hot textures, and strength of the gel.

### Experiments 21-25 Comparative formulations with 1% kappa-carrageenan and 4% plant proteins of various origins.

To verify the unique synergy between kappa-carrageenan and rubisco, experiments were carried out to assess potential interaction with carrageenan and other plant proteins for creation of vegan sausages were carried out according to the same preparation process and measurement procedure described in Examples 1-16.

Preset concentration of kappa-carrageenan for Experiments 21-25 is 1% w/w equivalent to formulation of Example 2.

### Composition (% by weight) of vegan cold cut products

| Ingredients | Experiment 21 | Experiment 22 | Experiment 23 | Experiment 24 | Experiment 25 |
|---|---|---|---|---|---|
| Water/Ice | 75.75 | 73.45 | 76.75 | 75.75 | 74.25 |
| Rapeseed Oil | 12 | 12 | 12 | 12 | 12 |
| Methylcellulose | 1.25 | 1.25 | 1.25 | 1.25 | 1.25 |
| Psyllium husk | 2 | 2 | 2 | 2 | 2 |
| NaCl | 2 | 2 | 2 | 2 | 2 |
| Faba bean protein | 2 | 2 | 2 | 2 | 6 |
| Kappa-Carrageen | 1 | 1 | 1 | 1 | 1 |
| Potato Protein | 4 | 0 | 0 | 0 | 0 |
| Canola Protein | 0 | 4 | 0 | 0 | 0 |
| Soy protein | 0 | 0 | 4 | 0 | 0 |
| Pea protein | 0 | 0 | 0 | 4 | 0 |

### pH and viscosity of emulsion before filling into casing

| | Experiment 21 | Experiment 22 | Experiment 23 | Experiment 24 | Experiment 25 |
|---|---|---|---|---|---|
| pH | 6.6 | 6.6 | 6.7 | 6.1 | 6.4 |
| Viscosity at 15°C (mPas) | 10862 | 10791 | 12898 | 13771 | 10267 |

### Texture assessment (Description and Hardness measurement)

| | Experiment 21 | Experiment 22 | Experiment 23 | Experiment 24 | Experiment 25 |
|---|---|---|---|---|---|
| Description of cold texture | Pasty | Pasty | Pasty | Pasty | Pasty |
| Hardness cold (15°C) [g] | 1598 | 1240 | 772 | 720 | 811 |
| SD Hardness cold (15°C) | 83 | 255 | 77 | 72 | 124 |
| Hardness hot (72°C) [g] | 1123 | 957 | 1095 | 1018 | 1132 |
| SD Hardness hot (72°C) | 52 | 111 | 70 | 29 | 94 |

Hardness of sausage measured at cold temperature is higher for the formulation of Example 2 compared to results of Experiments 21-25. Hardness measured at hot temperature is comparable for Example 2 and Experiments 21-25.

### Experiments 26-30 Comparative formulations with 2.5% kappa-carrageenan and 4% plant proteins of various origin

To verify the unique synergy between kappa-carrageenan and rubisco versus potential interaction with other plant proteins several experiments for creation of vegan sausages were carried out according to the same preparation process and measurement procedure described in Experiments 1-16.

### Composition (% by weight) of vegan cold cut products

| Ingredients | Experiment 26 | Experiment 27 | Experiment 28 | Experiment 29 | Experiment 30 |
|---|---|---|---|---|---|
| Water/Ice | 74.25 | 74.25 | 74.25 | 74.25 | 74.25 |
| Rapeseed Oil | 12 | 12 | 12 | 12 | 12 |
| Methylcellulose | 1.25 | 1.25 | 1.25 | 1.25 | 1.25 |
| Psyllium husk | 2 | 2 | 2 | 2 | 2 |
| NaCl | 2 | 2 | 2 | 2 | 2 |
| Faba bean protein | 2 | 2 | 2 | 2 | 6 |

| | | | | | |
|---|---|---|---|---|---|
| Kappa-carrageen | 2.5 | 2.5 | 2.5 | 2.5 | 2.5 |
| Potato Protein | 4 | 0 | 0 | 0 | 0 |
| Canola Protein | 0 | 4 | 0 | 0 | 0 |
| Soy protein | 0 | 0 | 4 | 0 | 0 |
| Pea protein | 0 | 0 | 0 | 4 | 0 |

### pH and viscosity of emulsion before filling into casing

| | Experiment 26 | Experiment 27 | Experiment 28 | Experiment 29 | Experiment 30 |
|---|---|---|---|---|---|
| pH | 6.7 | 6.7 | 6.7 | 6.2 | 6.5 |
| Viscosity at 15°C (mPas) | 14858 | 11573 | 18576 | 16659 | 14092 |

### Texture assessment (Description and Hardness measurement)

| | Experiment 26 | Experiment 27 | Experiment 28 | Experiment 29 | Experiment 30 |
|---|---|---|---|---|---|
| Description of cold texture | Moderate firm | Pasty | Pasty | Pasty | Pasty |
| Hardness cold (15°C) [g] | 2790 | 2532 | 1646 | 1587 | 1550 |
| SD Hardness cold (15°C) | 125 | 98 | 41 | 43 | 114 |
| Hardness hot (72°C) [g] | 1574 | 1855 | 1837 | 1559 | 1777 |
| SD Hardness hot (72°C) | 79 | 52 | 243 | 63 | 109 |

The hot and cold bite consistency as resembled by the measurement of gel hardness at different temperatures revealed that cold consistency is firmer for sausages produced by a formulation described in Example 3. Furthermore, gel hardness at hot temperature is higher for the formulation described in Example 3 compared to the formulation comprising konjac gum as wells as to various formulations comprising other sources of protein, namely potato protein, canola protein, soy protein isolate, pea protein isolate and faba bean protein isolate. The results clearly show that a combination of rubisco and kappa-carrageenan is unique in that it can replace potato protein and konjac gum or konjac glucomannan, producing a vegan emulsion-like product, e.g. a vegan sausage, with good cold and hot stabilities from a smaller number of ingredients.

### Example 4-6: Formulations for vegan cold cuts

The following experiment relates to compositions and a method for production of various vegan sausage types, in particular cold cuts.

### Composition (% by weight) of vegan cold cut products

| Ingredients | Example 4 | Example 5 | Example 6 |
|---|---|---|---|
| Water/Ice | 74.25 | 72.00 | 68.50 |
| Rapeseed Oil | 12 | 12 | 12 |
| Methylcellulose | 1.25 | 0 | 0 |
| Psyllium husk | 2 | 2 | 2 |
| NaCl | 2 | 2 | 2 |
| Faba bean protein | 2 | 2 | 2 |
| Carrageenan | 2.5 | 3.0 | 3.5 |
| Rubisco protein | 4 | 7 | 10 |

### pH of emulsion before filling into casing

| | Example 4 | Example 5 | Example 6 |
|---|---|---|---|
| pH | 6.7 | 6.9 | 7.2 |

### Texture assessment (Description and Hardness measurement)

| | Example 4 | Example 5 | Example 6 |
|---|---|---|---|
| Description of cold texture | Firm | Very firm | Very firm; but firmer than Ex. 5 |
| Hardness cold (15°C) [g] | 3781 | > 6000 | > 6000 |
| SD Hardness cold (15°C) | 132 | nd | nd |
| Hardness hot (72°C) [g] | 2402 | 936 | 1519 |
| SD Hardness hot (72°C) | 210 | 104 | 45 |

These examples show that without methylcellulose, very strong gels are obtainable, which are in particular useful for cold cuts such as sausages which are used cold, ham and the like.

### Example 7 various vegan sausages

The following experiment relates to compositions and a method for production of various vegan sausage types.

| Ingredients | Vegan salami-type sausage |
|---|---|
| Water/Ice | 69.25 |
| Rapeseed Oil | 10 |
| Methylcellulose | 1.25 |
| Psyllium husk | 2 |
| NaCl | 2 |
| Faba bean protein | 2 |
| Kappa-carrageenan | 2.5 |
| Rubisco protein | 4 |
| Spice mix (pepper, coriander, ginger, garlic) | 1.5 |
| Red beet extract | 0.5 |
| Tapioca starch particles (hydrated) | 5 |

For preparation of a salami, chorizo or pepperoni type sausage the following method was used: The manufacturing of the sausage dough is done in a high-speed bowl chopper that can also apply vacuum. Oil and dry ingredients are added to the bowl chopper and mixed (2000 rpm). Then water/ice 1:1 is added. The emulsion is blended for 3 min. to create the plant-based sausage dough. Sodium chloride, spice and coloring are added, and blending continued for a few rounds. The dough is blended under vacuum until a temperature of 10-12°C is reached. Tapioca starch particles (e.g. Tapiocaline VA 540 SG by Tipiak Epicerie SAS) for fat resemblance are pre-hydrated in excess of water and added to the dough. Blending is continued under vacuum for some rounds to guarantee even distribution of particles.

The created sausage dough is filled under vacuum into suitable casings of 40 mm diameter.

Sausages were cooked at 92°C for 1:45h to a core temperature of >85°C. Afterwards, sausages were cooled down in cold water to room temperature and stored in a fridge at 4°C.

Alternatively, to tapioca starch particles, it is also possible to add comminuted particles of a vegan fat replacer. Vegan fat replacers might be produced by combinations of water, oil and starch. Alternatively, such a system might also comprise alginate and/or methylcellulose. It may also be composed of other hydrocolloids that guarantee stability of contour of the individual particles within the sausage dough.

In order to mimic other types of sausages like e.g. coarse bratwurst it is also possible to incorporate hydrated and shredded TVP (textured vegetable protein) particles to resemble chunks of meat.

## Claims

1. Composition suitable for use in preparing meat analogue emulsion products comprising rubisco protein and kappa-carrageenan in relative amounts between 1:5 to 30:1.

2. Composition according to claim 1, wherein the composition comprises 0.5-30 wt% rubisco protein and 0.1-15 wt% kappa-carrageenan in the finished meat analogue product, preferably 1-15 wt% rubisco and 0.5-5 wt% kappa-carrageenan.

3. Composition according to any one of claims 1-2, wherein the composition further comprises methylcellulose, preferably in an amount of 0.1 - 15 wt%, relative to the finished meat analogue product.

4. Composition according to any one of claims 1-3, wherein the composition further comprises vegan protein other than rubisco preferably in an amount of 0.5 - 30 wt% relative to the meat analogue product.

5. Composition according to claim 4, wherein the vegan protein is selected from the group consisting of low to moderately soluble plant proteins, preferably potato, canola, faba bean, pea, soybean, wheat, chickpea, lupin, almond, mung bean, single cells (microalgae, yeast, bacteria, archaea), more preferably soy protein, pea protein, potato protein, canola protein and combination thereof

6. Composition according to any one of claims 1-5, wherein the composition further comprises a cold thickening agent, preferably a polysaccharide, preferably in an amount of 0.5 -30 wt% relative to the meat analogue product.

7. Composition according to any one of claims 1-6, wherein the composition further comprises one or more of salt, seasoning agents, structuring agents, preferably in an amount of 0.01 - 30 wt%.

8. Meat analogue comprising the composition of any one of claims 1-7.

9. Meat analogue according to claim 8, wherein the meat analogue comprises the composition according to 1-7 in an amount of between 2-40 wt%, preferably 5-30 wt% and further comprising water and a plant-based lipid.

10. Meat analogue according to claim 9 wherein the composition contains 30-90 wt% water and 2-40 wt% plant-based lipid, preferably 5-50 wt% lipid.

11. Meat analogue according to any one of claims 9-10 wherein the plant-based lipid comprises corn oil, palm oil, rapeseed oil, canola oil, sunflower oil, soybean oil, coconut oil, or mixtures thereof.

12. Meat analogue according to any one of claims 9-11, wherein the composition has a pH between 4-9 preferably between 5-8.

13. Meat analogue according to any one of claims 8-12 wherein the meat analogue is filled in a casing or container.

14. Meat analogue according to claim 13 wherein the meat analogue is a sausage or ham, such as frankfurters, like wiener, hot dogs, or cold cuts, like ham, pepperoni, salami, bologna, mortadella or bierwurst.

15. Meat analogue according to any one of claims 8-14, wherein the meat analogue does not comprise konjak gum or konjac galactomannan and/or methylcellulose and has the following emulsion characteristics:
∘ gel hardness at 15 °C of about 3000 g or higher
∘ gel hardness at 72 °C of about 2000 g or higher (with methylcellulose)
∘ gel hardness at 72 °C of about 900g or higher (without methylcellulose)
